# EUROPÄISCHE PATENTANMELDUNG

(11) **EP 3 246 006 A1**
(43) Veröffentlichungstag der Anmeldung: **22.11.2017**
(21) Anmeldenummer: 16170189.1
(22) Anmeldetag: 18.05.2016
(51) Int. Cl.: A61K 8/46, A61Q 5/02, C11D 1/28, C11D 1/37, C11D 3/00, C11D 11/00

(54) **WÄSSRIGE TENSID-ZUSAMMENSETZUNGEN**

(71) Anmelder: BASF SE, 67056 Ludwigshafen am Rhein (DE)
(72) Erfinder: Brunn, Claudia, 40597 Duesseldorf (DE); Behler, Ansgar, 46240 Bottrop (DE); Raths, Hans-Christian, 40789 Monheim (DE); Stanislowski, Detlev, 40822 Mettmann (DE); Hermanns, Gerhard, 42699 Solingen (DE)
(74) Vertreter: BASF IP Association

(57) **Zusammenfassung**

Vorgeschlagen werden wässrige Tensid-Zusammensetzungen enthaltend
• ein oder mehrere **alpha-Sulfofettsäuredisalze** (A) der allgemeinen Formel (I),

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
• ein oder mehrere Sulfoketone (B), die ausgewählt sind aus den Verbindungen (F) und den Verbindungen (G),
wobei die Verbindungen (F) die allgemeine Formel (VI)

R⁶CH₂-CO-CHR⁷(SO₃M⁸) (VI),

aufweisen, worin die Reste R6 und R7 - unabhängig voneinander - einen linearen oder verzweigten Alkylrest mit 6 bis 18 C-Atomen bedeuten und der Rest M8 ausgewählt wird aus der Gruppe H, Li, Na, K, Ca/2, Mg/2, Ammonium und Alkanolamine,
und wobei die Verbindungen (G) die allgemeine Formel (VII)

(SO₃M⁹)R⁸CH-CO-CHR⁹(SO₃M¹⁰) (VII),

aufweisen, worin die Reste R8 und R9 - unabhängig voneinander - einen linearen oder verzweigten Alkylrest mit 6 bis 18 C-Atomen bedeuten und die Reste M9 und M10 - unabhängig voneinander - ausgewählt werden aus der Gruppe H, Li, Na, K, Ca/2, Mg/2, Ammonium und Alkanolamine, und
**• Wasser,**
wobei die Menge der Verbindungen (A) größer ist als die Menge der Verbindungen (B) - beides bezogen auf die gesamte wässrige Tensid-Zusammensetzung - und wobei im Hinblick auf sogenannte Estersulfonate eine in den Patentansprüchen näher definierte Randbedingung einzuhalten ist. Die Zusammensetzungen weisen ein gutes Schaumvermögen und eine angenehme Sensorik des Schaumes sowie eine gute Hautverträglichkeit auf und eignen sich für kosmetische Mittel sowie Wasch- und Reinigungsmittel.

## Beschreibung

### Gebiet der Erfindung

Die vorliegende Erfindung betrifft wässrige Tensid-Zusammensetzungen mit einem Gehalt an alpha-Sulfofettsäuredisalzen sowie speziellen Sulfoketonen.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können.

### Beschreibung der Erfindung

Die Aufgabe der vorliegenden Erfindung hat darin bestanden, wässrige Tensid-Zusammensetzungen bereitzustellen, die sich durch die im Folgenden genannten Eigenschaften auszeichnen:
- Gutes Schaumvermögen.
- Angenehme Sensorik des Schaumes.
- Gute Hautverträglichkeit.

Gegenstand der Erfindung sind zunächst wässrige Tensid-Zusammensetzungen enthaltend
- ein oder mehrere **alpha-Sulfofettsäuredisalze** (**A**) der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Sulfoketone** (**B**), die ausgewählt sind aus den Verbindungen (F) und den Verbindungen (G),
   wobei die Verbindungen (F) die allgemeine Formel (VI)

   R⁶CH₂-CO-CHR⁷(SO₃M⁸) (VI),

   aufweisen, worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine,
   und wobei die Verbindungen (G) die allgemeine Formel (VII)

   (SO₃M⁹)R⁸CH-CO-CHR⁹(SO₃M¹⁰) (VII),

   aufweisen, worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine, und
- **Wasser**,
   wobei folgende Maßgaben gelten:
- Die Menge der Verbindungen (A) ist größer als die Menge der Verbindungen (B) - beides bezogen auf die gesamte wässrige Tensid-Zusammensetzung.
- Sofern die wässrigen Tensid-Zusammensetzungen ein oder mehrere Estersulfonate (E) der allgemeinen Formel (V),

   R²CH(SO₃M⁷)COOR³ (V)

   worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform gilt die Maßgabe, dass der Anteil der Verbindungen (A) in den wässrigen Tensid-Zusammensetzungen, bei denen der Rest R¹ ein Alkenylrest ist, - bezogen auf die Gesamtmenge der Verbindungen (A) - bei 3 Gew.-% oder weniger liegt.

In einer bevorzugten Ausführungsform bedeutet der Rest R¹ in der Formel (I) einen gesättigten, linearen Alkylrest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 70 Gew.-% oder mehr und vorzugsweise bei 90 Gew.-% oder mehr liegt.

In einer Ausführungsform werden die Reste M¹ und M² in der Formel (I) ausgewählt aus der Gruppe H (Wasserstoff) und Na (Natrium).

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten im Kapitel "Bevorzugte Ausführungsformen" beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.-%,
- der Gehalt an (C) liegt im Bereich von 0 bis 20 Gew.-%,
- der Gehalt an (D) liegt im Bereich von 0 bis 20 Gew.-%,
mit der Maßgabe, dass die Summe der Komponenten (A), (C) und (D) in dieser Mischung 100 Gew.-% beträgt.

### Zu den Verbindungen (B)

Wie oben ausgeführt enthalten die erfindungsgemäßen wässrigen Tensid-Zusammensetzungen neben den Verbindungen (A) und Wasser ein oder mehrere **Sulfoketone (B)**, die ausgewählt werden aus den Verbindungen (F) und (G).

Die **Verbindungen (F)** haben die allgemeine Formel (VI)

R⁶CH₂-CO-CHR⁷(SO₃M⁸) (VI),

worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (F) werden im Rahmen der vorliegenden Erfindung als Mono-Sulfo-Ketone bezeichnet.

In einer bevorzugten Ausführungsform bedeuten die Reste R⁶ und R⁷ in der Formel (VI) - unabhängig voneinander - einen gesättigten, linearen Rest mit 10 bis 16 C-Atomen, wobei in Bezug auf die Verbindungen (F) gilt, dass der Anteil der Verbindungen (F), bei denen die Reste R⁶ und R⁷ einen Decyl- und/oder ein Dodecylrest bedeuten, - bezogen auf die Gesamtmenge der Verbindungen (F) - bei 70 Gew.-% oder mehr und vorzugsweise bei 90 Gew.-% oder mehr liegt. In einer Ausführungsform wird der Rest M⁸ in der Formel (VI) ausgewählt aus der Gruppe H und Na.

Die **Verbindungen (G)** haben die allgemeine Formel (VII)

(SO₃M⁹)R⁸CH-CO-CHR⁹(SO₃M¹⁰) (VII),

worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (G) werden im Rahmen der vorliegenden Erfindung als Di-Sulfo-Ketone bezeichnet.

In einer bevorzugten Ausführungsform bedeuten die Reste R⁸ und R⁹ in der Formel (VII) - unabhängig voneinander - einen gesättigten, linearen Rest mit 10 bis 16 C-Atomen, wobei in Bezug auf die Verbindungen (G) gilt, dass der Anteil der Verbindungen (G), bei denen die Reste R⁸ und R⁹ einen Decyl- und/oder ein Dodecylrest bedeuten, - bezogen auf die Gesamtmenge der Verbindungen (G) - bei 70 Gew.-% oder mehr und vorzugsweise bei 90 Gew.-% oder mehr liegt. In einer Ausführungsform werden die Reste M⁹ und M¹⁰ in der Formel (VII) ausgewählt aus der Gruppe H und Na.

Die Herstellung der Verbindungen (F) und (G) unterliegt keinen besonderen Einschränkungen und sie können nach allen dem Fachmann bekannten Methoden hergestellt werden.

In einer Ausführungsform werden die Verbindung (F) und (G) durch Sulfonierung entsprechender Ketone mit gasförmigem Schwefeltrioxid hergestellt, wie in der deutschen Offenlegungsschrift DE-A-42,20,580 beschrieben.

In einer anderen Ausführungsform geht man zur Herstellung der Verbindungen (F) und (G) von Fettsäuren aus. Dabei führt man die Sulfierung von flüssigen Fettsäuren mit gasförmigem Schwefeltrioxid so durch, dass dabei neben Disalzen (A) auch die Verbindungen (F) und (G) entstehen, was dadurch realisiert werden kann, dass man die Sulfierung wie folgt durchführt: Das Verhältnis der Rohstoffe Fettsäure, die auch in Form von Gemischen von Fettsäuren unterschiedlicher Kettenlänge eingesetzt werden können, und Schwefeltrioxid wird so eingestellt, dass man 1,0 bis 1,5 mol und insbesondere 1,0 bis 1,25 mol SO₃ pro mol Fettsäure(n) einsetzt. Die Fettsäuren werden dabei mit einer Vorlagetemperatur im Bereich von 70 bis 100 °C in den Reaktor eingebracht. Nach der Sulfierung wird das erhaltene flüssige Sulfierprodukt in einer temperierten Nachreaktionsschlange für 5 bis 20 Minuten auf dieser Temperatur gehalten und gealtert. Anschließend erfolgt die Neutralisation mit einer wässrigen Base, vorzugsweise Natriumhydroxid, in der Regel bei einem pH-Wert im Bereich von von 5 bis 10, insbesondere von 5 bis 7. Im Anschluss kann eine saure Bleiche - der pH wird hierbei auf einen Wert von 7 oder weniger eingestellt - mit Wasserstoffperoxid durchgeführt werden.

### Bevorzugte Ausführungsformen

In einer Ausführungsform enthalten die erfindungsgemäßen wässrigen Tensid-Zusammensetzungen neben den Verbindungen (A), (B) und Wasser zusätzlich ein oder mehrere **Verbindungen** (C) der allgemeinen Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform enthalten die erfindungsgemäßen wässrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

In einer bevorzugten Ausführungsform enthalten die erfindungsgemäßen wässrigen Tensid-Zusammensetzungen die Verbindungen (A), (B), (C), (D) sowie Wasser. Besonders bevorzugt sind dabei wässrige Tensid-Zusammensetzungen, die die Verbindungen (A), (C), (D), (F) und (G) enthalten. Dabei ist es besonders bevorzugt, wenn die Reste M¹ und M² der Verbindungen (A), der Rest M⁵ der Verbindungen (C), der Rest M⁶ der Verbindungen (D), der Rest R⁸ der Verbindungen (F) und die Reste M⁹ und M¹⁰ der Verbindungen (G) ausgewählt werden aus der Gruppe H und Na.

In einer Ausführungsform enthalten die erfindungsgemäßen Zusammensetzungen neben den Verbindungen (A), (B), (C), (D) und Wasser zusätzlich ein oder mehrere Verbindungen (H), die ausgewählt sind aus der Gruppe der Alkylglycoside (H1), Amidoalkylbetaine (H2) und der N-Acyl-Glutaminsäure-Verbindungen (H3).

Die **Verbindungen (H1)**, die im Rahmen der vorliegenden Erfindung als **Alkylglycoside** bezeichnet werden, haben die Formel (Ha),

R¹⁰O-[G]ₚ (IIa)

in der R² für einen Alkyl- und/oder Alkenylrest mit 8 bis 18 C-Atomen, G für einen Zuckerrest mit 5 oder 6 Kohlenstoffatomen und p für Zahlen zwischen 1 und 10 steht. In Bezug auf die Verbindungen (H1) gilt ferner die Maßgabe, dass der Anteil der Verbindungen (H1), bei denen der Rest R² ein Alkyl- oder Alkenylrest mit 15 oder mehr C-Atomen ist, - bezogen auf die Gesamtmenge der Verbindungen (H1) in den wässrigen Tensid-Zusammensetzungen - bei 5 Gew.-% oder weniger liegt.

Ausdrücklich sei festgestellt, dass die Bezeichnung der Verbindungen (H1) als Alkylglykoside - fortan auch als APGs (Singular: APG) bezeichnet - lediglich einer sprachlich einfachen Bezeichnung der Verbindungen (H1) dient und nicht strukturell einschränkend zu verstehen ist; denn in der formelmäßigen Definition der Verbindungen (H1) ist klargestellt, dass der Rest R¹⁰ sowohl einen Alkyl- als auch einen Alkenylrest bedeuten kann und ferner - dies zeigt der Index p - dass es sich um Alkyl- bzw. Alkenyloligoglykoside handelt.

APGs der hier beanspruchten Form können nach den einschlägigen Verfahren der präparativen organischen Chemie erhalten werden. Die APGs können sich von Aldosen bzw. Ketosen mit 5 oder 6 C-Atomen ableiten. Vorzugsweise leiten sich die APGs von Glucose ab.

Die Indexzahl p in der allgemeinen Formel (IIa) gibt den Oligomerisierungsgrad (DP-Grad = degree of polymerization) an. Der Oligomerisierungsgrad der APGs liegt zwischen 1 und 10 und vorzugsweise zwischen 1 und 6. Während p in einem einzelnen APG-Molekül stets ganzzahlig ist und hier vor allem die Werte im Bereich von 1 bis 6 annimmt, ist der Wert p für ein APG, das eine Mischung verschiedener APG-Moleküle darstellt, die sich in den je individuellen p-Werten unterscheiden, eine analytisch ermittelte rechnerische Größe, die meistens eine gebrochene Zahl darstellt. Vorzugsweise werden APGs mit einem mittleren Oligomerisierungsgrad p im Bereich von 1,1 bis 3,0 eingesetzt. Dabei sind insbesondere solche APGs bevorzugt, deren mittlerer Oligomerisierungsgrad kleiner als 2 ist und vorzugsweise im Bereich von 1,1 bis 1,8 und insbesondere im Bereich von 1,2 bis 1,7 liegt.

Der mittlere Oligomerisationsgrad ist dabei in dem Sinne zu verstehen, wie er in der Monographie K. Hill, W. von Rybinski, G. Stoll "Alkyl Polyglycosides. Technology, Properties and Applications" (VCH-Verlagsgesellschft, 1996) im Abschnitt "Degree of polymerization" (vergleiche die Seiten 11-12 des Buches) definiert ist: Dort heißt es "The average number of glycose units linked to an alcohol group is described as the (average) degree of polymerization (DP)." In der erläuternden Figur 2, die eine typische Verteilung von Dodecylglycosid Oligomeren eines AOPGs mit einem DP-Grad von 1,3 beschreibt, ist der mittlere DP-Grad auch durch eine entsprechende mathematische Formel beschrieben.

Der Rest R¹⁰ leitet sich vorzugsweise von primären Alkoholen mit 4 bis 11 C-Atomen und vorzugsweise 8 bis 10 C-Atomen ab. Typische Beispiele für geeignete Reste R¹⁰ sind Butyl-, Hexyl-, Octyl-, Decyl-, Undecyl-, Dodecyl- und Myristyl-. Sie leiten sich von den gesättigten Fettalkoholen Butanol-1, Capronalkohol (Hexanol-1), Caprylalkohol (Octanol-1), Caprinalkohol (Decanol-1), Undecanol-1, Lamylalkohol (Dodecanol-1) und Myristylalkohol (Tetradecanol-1) ab, wie sie beispielsweise bei der Hydrierung von technischen Fettsäuremethylestern oder im Verlauf der Hydrierung von Aldehyden bei der Roelen'schen Oxosynthese erhalten werden.

Bevorzugt sind APGs, die sich von Glucose ableiten und bei denen der Rest R¹⁰ ein gesättigter Alkylrest mit 8 bis 12 C-Atomen ist und die einen mittleren Oligomerisierungsgrad im Bereich von 1,1 bis 3 und insbesondere im Bereich von 1,2 bis 1,8 und besonders bevorzugt im Bereich von 1,2 bis 1,7 aufweisen. Diese APGs können beispielsweise dadurch hergestellt werden, dass man einen Zucker, insbesondere Glucose, säurekatalysiert mit einem Fettalkoholgemisch umsetzt, wobei man als Fettsäuregemisch vorzugsweise einen bei der destillativen Auftrennung von technischem C₈₋₁₈-Kokosfettalkohol anfallenden Vorlauf einsetzt, der überwiegend Octanol-1 und Decanol-1 sowie geringe Mengen Dodecanol-1 enthält.

Die **Verbindungen (H2)**, die im Rahmen der vorliegenden Erfindung als **Amidoalkylbetaine** bezeichnet werden, haben die Formel (IIb)

R¹¹-CO-NH-(CH₂)_{y}-N⁺(CH₃)₂-CH₂-COO⁻ (IIb)

worin der Rest R¹¹ einen linearen oder verzweigten Alkyl- oder Alkenylrest mit 7 bis 19 C-Atomen bedeutet und der Index y eine ganze Zahl im Bereich 2 bis 4 ist. Ferner gilt - wie ebenfalls oben angegeben - die Maßgabe, dass der Anteil der Verbindungen (H2), bei denen der Rest R² ein Alkenylrest ist, - bezogen auf die Gesamtmenge der Verbindungen (H2) in den wässrigen Tensid-Zusammensetzungen - bei 3 Gew.-% oder weniger liegt.

Die Verbindungen (H2) können nach allen dem Fachmann einschlägig bekannten Methoden hergestellt werden.

In einer Ausführungsform bedeutet der Index y in der Formel (IIb) die Zahl 3.

In einer Ausführungsform bedeutet Rest R¹¹ in der Formel (IIb) einen gesättigten, linearen Rest mit 11 bis 17 C-Atomen, wobei in Bezug auf die Verbindungen (H2) gilt, dass der Anteil der

Verbindungen (H2), bei denen der Rest R¹¹ ein Undecyl- oder ein Tridecylrest ist, - bezogen auf die Gesamtmenge der Verbindungen (H2) - bei 60 Gew.-% oder mehr liegt.

In einer bevorzugten Zusammensetzung handelt es sich in Bezug auf die Verbindungen (H2) um Kokosamidopropylbetain. Es handelt sich um ein technisch verfügbares Produkt, das typischerweise in zwei Schritten hergestellt wird:
Zunächst setzt man Kokosfettsäure mit Dimethylaminopropylamin (DMAPA, chemische Formel NH₂-(CH₂)₃-N(CH₃)₂) um. Das dabei resultierende Amid wird dann in einem zweiten Schritt mit Natriumchloracetat (chemische Formel Cl-CH₂-COONa) in Gegenwart von NaOH umgesetzt, wobei unter Abspaltung von NaCl eine Quarternierung erfolgt. Das so erhältliche Produkt technischer Qualität kann neben Kokosamidopropylbetain und NaCl herstellbedingt als Nebenprodukte Glycerin, Partialglyceride, Glykolsäure, Diglykolsäure und freie Fettsäure enthalten, wobei sich der Gehalt dieser Nebenprodukte durch die Wahl geeigneter Herstellbedingungen vermindern lässt. Gewünschtenfalls können diese Nebenprodukte auch durch zusätzliche Reinigungsschritte Aufreinigung in ihrem Gehalt weiter reduziert oder ganz eliminiert werden.

Die Verbindungen (H3), die im Rahmen der vorliegenden Erfindung als **N-Acyl-Glutaminsäure-Verbindungen** bezeichnet werden, haben die Formel (IIc)

M³OOC-CH₂-CH₂-CH(NH-CO-R¹²)-COOM⁴ (IIc)

worin der Rest R¹² einen linearen oder verzweigten Alkyl- oder Alkenylrest mit 7 bis 19 C-Atomen bedeutet und die Reste M³ und M⁴ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin. In einer Ausführungsform werden die Reste M³ und M⁴ ausgewählt aus der Gruppe H und Na.

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A), (B), (D), (E), (F) oder (G) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

## Patentansprüche

1. Wässrige Tensid-Zusammensetzungen enthaltend
• ein oder mehrere **alpha-Sulfofettsäuredisalze** (**A**) der allgemeinen Formel (I),
R¹CH(SO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamin,
• ein oder mehrere **Sulfoketone** (B), die ausgewählt sind aus den Verbindungen (F) und den Verbindungen (G),
wobei die Verbindungen (F) die allgemeine Formel (VI)
R⁶CH₂-CO-CHR⁷(SO₃M⁸) (VI),
aufweisen, worin die Reste R6 und R7 - unabhängig voneinander - einen linearen oder verzweigten Alkylrest mit 6 bis 18 C-Atomen bedeuten und der Rest M8 ausgewählt wird aus der Gruppe H, Li, Na, K, Ca/2, Mg/2, Ammonium und Alkanolamine,
und wobei die Verbindungen (G) die allgemeine Formel (VII)
(SO₃M⁹)R⁸CH-CO-CHR⁹(SO₃M¹⁰) (VII),
aufweisen, worin die Reste R8 und R9 - unabhängig voneinander - einen linearen oder verzweigten Alkylrest mit 6 bis 18 C-Atomen bedeuten und die Reste M9 und M10 - unabhängig voneinander - ausgewählt werden aus der Gruppe H, Li, Na, K, Ca/2, Mg/2, Ammonium und Alkanolamine, und
• **Wasser**,
wobei folgende Maßgaben gelten:
• Die Menge der Verbindungen (A) ist größer als die Menge der Verbindungen (B) - beides bezogen auf die gesamte wässrige Tensid-Zusammensetzung.
• Sofern die wässrigen Tensid-Zusammensetzungen ein oder mehrere **Estersulfonate (E)** der allgemeinen Formel (V),
R²CH(SO₃M⁷)COOR³ (V)
worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr vorliegen müssen.

2. Zusammensetzungen nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Zusammensetzungen nach Anspruch 1 oder 2, wobei die Reste M¹ und M² sowie die Reste M⁸, M⁹ und M¹⁰ ausgewählt werden aus der Gruppe H (Wasserstoff) und Na (Natrium).

4. Zusammensetzungen nach einem der Ansprüche 1 bis 3, wobei die Zusammensetzungen zusätzlich ein oder mehrere **Verbindungen (C)** der allgemeinen Formel (III)
R⁴COOM⁵ (III)
enthalten, worin der Rest R⁴ einen linearen oder verzweigten Alkyl- oder Alkenylrest mit 7 bis 19 C-Atomen bedeutet und der Reste M⁵ ausgewählt wird aus der Gruppe H, Li, Na, K, Ca/2, Mg/2, Ammonium und Alkanolamin.

5. Zusammensetzungen nach einem der Ansprüche 1 bis 4, wobei die Zusammensetzungen zusätzlich ein oder mehrere **anorganische Salze der Schwefelsäure (D)** der allgemeinen Formel (IV)
(M⁶)₂SO₄ (IV)
enthalten, wobei M⁶ ausgewählt wird aus der Gruppe Li, Na, K, Ca/2, Mg/2, Ammonium und Alkanolamin.

6. Verwendung der Zusammensetzungen nach einem der Ansprüche 1 bis 5 für kosmetische Mittel sowie Wasch- und Reinigungsmittel.

7. Verwendung der Zusammensetzungen nach einem der Ansprüche 1 bis 5 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

8. Verwendung der Zusammensetzungen nach einem der Ansprüche 1 bis 5 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.
